# EUROPEAN PATENT SPECIFICATION

(11) **EP 0 595 798 B1**
(45) Date of publication and mention of the grant of the patent: **03.03.1999**
(21) Application number: 90917241.3
(22) Date of filing: 18.10.1990
(51) Int. Cl.: C12P 21/08, C12N 5/08, A61K 35/14

(54) **BISPECIFIC HETEROANTIBODIES WITH DUAL EFFECTOR FUNCTIONS**
BISPEZIFISCHE HETEROANTIKÖRPER MIT ZWEIFACHEN EFFEKTORFUNKTIONEN
HETERO-ANTICORPS BISPECIFIQUES AVEC FONCTIONS EFFECTRICES DOUBLES

(30) Priority: 20.10.1989 US 424540
(43) Date of publication of application: 11.05.1994
(73) Proprietor: MEDAREX, INC., West Lebanon, NH 03784 (US)
(72) Inventor: FANGER, Michael, W., Lebanon, NH 03766 (US); GUYRE, Paul, M., Hanover, NH 03755 (US); BALL, Edward, D., Wexford, Pennsylvania 15090 (US)
(74) Representative: Price, Vincent Andrew
(86) International application number: US9005981
(87) International publication number: WO9105871

(56) References cited:
- WO-A-88/00052
- GB-A- 2 215 046
- JOURNAL OF IMMUNOLOGY, vol. 137, no. 11, 01 December 1986, American Association of Immunologists, Baltimore, MD (US); L. SHEN et al., pp. 3378-3382#
- JOURNAL OF IMMUNOLOGY, vol. 143, no. 5, 01 September 1989, American Association of Immunologists, Baltimore, MD (US); P.M. GUYRE et al., pp. 1650-1655#
- J.Exp.Med, vol.160, p.1686-1701, (1984)
- EPO Board of Appeal decision T 290/86
- EPO Board of Appeal decision T 958/90

## Description

### Background

The production of heteroantibodies for targeting effector cells comprising an antibody specific for the high affinity FcRI receptor linked to a second antibody specific for an antigen present on a target cell has been described. See, for example, Segal et al. , U.S. Patent Number 4,676,980; and Karpovsky et al. , J. Exp. Med. 160:1686-1701 (1984). Such constructs can be used to specifically kill unwanted cells (e.g. tumor cells or virus infected cells).

Recently, a monoclonal antibody has been produced which reacts with the high affinity Fc-gamma receptor through its variable region. Serum immunoglobulin does not compete with the antibody for binding to the Fc receptor. See, for example, Application; Anderson et al. , J. Biol. Chem. 261:12856 (1986); and Shen et al, J. Immunol. 137: 3378-3382 (1986). Consequently, serum IgG does not interfere with targeted effector cell killing. WO 88/00052 discloses heteroantibodies comprising an antibody, which binds to the high affinity Fcγ-receptor of monocytes, and an antibody which binds to a cell surface antigen of a target cell, eg. a tumour cell.

### Summary of the Invention

This invention pertains to the subject matter of the claims.

In a preferred embodiment, the antibody specific for the cell surface antigen of the target cell is an IgM molecule. Heteroantibodies formed with IgM can induce complement-mediated, as well as effector-cell-mediated, lysis of the target cell.

The heteroantibodies can be used to target and destroy unwanted cells such as tumor cells or virus infected cells. For this purpose, they can be administered alone or they can be pre-attached to effector cells for administration to a patient. They can also be used in conjunction with other molecules. For example, molecules of this invention can be used with cytokines such as interferon-γ which can activate or enhance their therapeutic potential.

### Detailed Description of the Invention

The heteroantibodies have at least two distinct binding specificities. The molecules contain an antibody or fragment thereof specific for a surface antigen of a target cell and an antibody or fragment thereof specific for the high affinity Fcγ receptor of effector cells. In addition, the heteroantibodies have dual effector functions. The heteroantibody is capable of inducing complement-mediated cell lysis and antibody-dependent cell mediated cytolysis.

The Fc-receptor binding specificity is provided by a binding agent which binds to the high affinity (p72) Fcγ receptor (FcRI) for human IgG without being blocked by human IgG. The preferred Fcγ receptor binding agent is an antibody, antibody fragment, antibody variable region, or genetic construct having the following characteristics:
a. it reacts specifically with the high affinity Fcγ receptor;
b. it reacts with the receptor through its antigen combining region independent of any Fc portion;
c. it reacts with an epitope of Fcγ receptor which is distinct from the Fc binding (i.e. ligand binding) site of the receptor; and
d. it binds ligand-occupied receptor.

The anti-Fcγ receptor antibodies of this invention can be produced as described in WO 88/00052 Fanger et al., "Monoclonal Antibodies to Fc Receptors for Immunoglobulin G on Human Mononuclear Phagocytes" A hybridoma producing a preferred antibody having the above characteristics, mAb 32.2, is available from the American Type Culture Collection (ATCC accession number HB 9469).

The target cell specificity and the complement-mediated cell lysis effector function is provided by an antibody specific for a surface antigen of the target cell. In a preferred embodiment, this antibody is an antibody which can direct complement-mediated cell lysis and provide the heteroantibody with this effector function. Preferably, the antibody specific for the target cell is an IgM. Heteroantibodies containing antibodies of this class demonstrate enhanced ability to kill targeted cells as is demonstrated in the Example which follows.

Target cells are cells whose elimination would be beneficial to the host. One important type of target cell is a tumor cell. Heteroantibody of this invention can have specificity for FcRI and specificity for a tumor-associated or tumor specific antigen.

Antibodies with a desired tumor specificity for production of heteroantibody can be produced or can be selected from available sources. Monoclonal antibodies against tumor-associated antigens can be made by the methods of Koprowski et al. , U.S. Patent 4,172,124. Many suitable anti-tumor antibodies are presently available.

Specific anti-tumor antibodies would include, but not be limited to:

| Antibody | Specificity |
|---|---|
| AML-2-23, PM-81, PMN-6, PMN-19 | Myeloid Leukemia |
| SCCL-1, SCCL-175 | Small Cell Lung Carcinoma |
| OC125, OVCT-3 | Ovarian Carcinoma |
| COL-1, COL-2,...COL-13 | Colon Carcinoma |

A preferred anti-tumor antibody is an antibody specific for the CD15 antigen as represented by the antibody designated PM-81 in the above table. The CD15 antigen is expressed by colon and breast tumor cells in addition to myeloid leukemia cells (as indicated in the table).

In addition to tumor cells, the effector cell can be targeted against auto-antibody producing lymphocytes for treatment of autoimmune disease or an IgE-producing lymphocyte for treatment of allergy. The target can also be a microorganism (bacterium or virus) or a soluble antigen (such as rheumatoid factor or other auto-antibodies).

Bivalent heteroantibodies comprise an antibody (or fragment) specific for Fcγ receptor, coupled to an antibody (or fragment) specific for a cell surface antigen of a target cell. Heteroantibodies can be prepared by conjugating Fcγ receptor antibody with antibody specific for the target cell antigen as is described in detail in the Example below. A variety of coupling or crosslinking agents can be used to conjugate the antibodies. Examples are protein A, carboiimide, dimaleimide, dithio-bis-nitrobenzoic acid (DTNB), and N-succinimidyl-3-(2-pyridyldithio) propionate (SPDP). SPDP and DTNB are the preferred agents; procedures for crosslinking antibodies with these agents are known in the art. See e.g., Karpovsky, B. et al., (1984) J. Exp. Med. 160:1686; Liu, M.A. et al., (1985) Proc. Natl. Acad. Sci USA 82:8648; Segal, D.M. and Perez, P., U.S. Patent No. 4,676,980 (June 30, 1987); and Brennan, M. Biotechniques 4:424 (1986).

Heteroantibodies can be administered to target the killing of unwanted cells in two general ways. The molecules can be given in free form. Alternatively, the molecules can be attached to the surface of effector cells in vitro and the cells can be administered. In each mode the principle is the same; the effector cell is targeted toward the cell bearing the targeted antigen.

Effector cells for targeting are human leukocytes, preferably macrophages. Other cells can include monocytes, activated neutrophils, and possibly activated natural killer (NK) cells and eosinophils. Macrophages can be treated with IFN-γ before targeting to increase the number of Fc receptors for attachment of the targeting antibody or heteroantibody. Neutrophils and NK cells can also be activated with IFN-γ in this way. The effector cells may also be activated before targeting by other cytokines such as tumor necrosis factor, lymphotoxin, colony stimulating factor, and interleukin-2. If desired, effector cells for targeting can be obtained from the host to be treated, or any other immunologically-compatible donor.

The targeted effector cells can be administered as a suspension of cells in a physiologically acceptable solution. The number of cells administered can be in the order of 10⁸-10⁹, but will vary depending on the therapeutic purpose. In general, the amount will be sufficient to obtain localization of the effector cell at the target cell, and to effect killing of the cell by complement mediated cell lysis and antibody dependent cell-mediated cytolysis (ADCC) and/or phagocytosis. Routes of administration can also vary. The targeted effector cells could be administered intravenously, intramuscularly, or intraperitoneally.

Heteroantibodies link antigen-specific binding agents to FcγR on effector cells in such a way that the large excess of human IgG in vivo does not interfere with binding of the molecule to effector cells or interfere with functioning of effector cells. This is possible because the anti-FcγR component of these molecules binds to FcγR at an epitope outside of its ligand binding domain. Effector cells (i.e. macrophages) targeted in this way can be employed to bring about antibody-dependent cell-mediated killing of HIV or HIV-infected cells.

The heteroantibodies have a potentially long half-life in vivo. This can result from the interaction of these constructs with FcγR on all monocytes and macrophages where it might remain for long periods of time, much of it out of circulation, but functionally active throughout the body on all cells of the reticuloendothelial system.

The invention is illustrated further by the following example.

### EXAMPLES

### Antibodies and Antibody Fragments.

The development and properties of mAb 32.2, a mouse mAb to the human monocyte high affinity Fc receptor, have been reported (Anderson, C.L. et al. (1986) J. Biol. Chem. 261:12856). Briefly, FcRI was isolated from U937 cells by affinity chromatography on immobilized human IgG and was injected into BALB/c mice. Five days after the last immunization, the splenocytes were fused with cells of the NS1 myeloma cell line. Supernatants of the hybrids were screened for their reactivity with U937 cells by an indirect immunofluorescence assay using a flow cytometer.

Selected hybrids cloned by limiting dilution, were rescreened and expanded. An IgG1 mAb was then selected that exhibited specific binding to the same 72,000 dalton protein (FcRI) precipitated by Sepharose-human IgG. This identity of reaction was shown by preclearing experiments and by identical isoelectric focussing patterns. Binding of mAb 32.2 to FcRI was independent of the Fc region of the antibody inasmuch as Fab' fragments of this mAb affinity adsorbed FcRI. The binding of both mAb 32.2 and human IgG1 to the intact U937 cell were not reciprocally inhibitory, indicating that mAb 32.2 does not interfere with the ligand binding site of FcRI. The IgG fraction of ascites fluid from pristane-primed mice injected with the 32.2 hybridoma was obtained by precipitation with 40% saturated ammonium sulfate. Ion exchange high pressure liquid chromatography (HPLC) with the use of a protein-pak 5PW DEAE column (Waters Chromatography Division, Millipore, Milford, MA) was used to purify the 32.2 IgGl antibody. The F(ab')₂ fragment was made according to the method of Parham (Parham, P. (1983) J. Immunol. 131:2895) by pepsin digestion at pH 3.5. Digestions were monitored by HPLC to ensure complete cleavage. F(ab')₂ fragments were purified by HPLC gel filtration chromatography by using a Bio-Sil® TSK 250 column (Bio-Rad, Richmond, CA), and Fab fragments were obtained by reduction with 1 mM dithiothreitol for 2 hr at 18°C, followed by alkylation with 2 mM iodoacetamide for 1 hr at 18°C.

### Heteroantibody Formation.

Heteroantibodies of Fab 32.2 plus mAb PM81 were made by the method of Karpovsky et al. (Karpovsky, B. (1984) J. Exp. Med. 160:1686). Fab 32.2 (or Fab W6/32) and mAb PM81 (at 1 to 3 mg/ml) were treated separately with an eightfold molar excess of the bifunctional reagent N-succinimidyl-3-(2 pyridyldithiol) propionate (SPDP) (Pharmacia, Uppsala, Sweden) for 2 hr at 18°C. SPDP-treated Fab 32.2 was dialyzed in phosphate-buffered saline (PBS), pH 7.4. SPDP-treated mAb PM81 was dialyzed in 0.1 M phosphate-0.1 M acetate-0.1 M NaCl, pH 4.5, was treated with 0.02 M dithiothreitol (30 min. 18°C), and was passed through a G-25 Sephadex® column (Pharmacia) equilibrated in 0.1 M phosphate, 0.1 M NaCl, pH 7.5. Equimolar amounts of the Fab 32.2 and mAb PM81 were then mixed and incubated at 18°C for 4 hr, after which cross-linking was terminated with 1 mM iodoacetamide. Heteroantibodies were dialyzed into PBS and were sterilized by 0.2 µm filtration. Preparations contained less than 15% uncross-linked Fab, and were at a concentration of 0.7 to 1.5 OD₂₈₀ U per ml.

### Effector Cells.

U937 cells obtained from the ATCC (Sundstrom C., and K. Nilsson (1976) Int. J. Cancer 17:565) were cultured in RPMI containing 10% heat-inactivated fetal bovine serum (FBS) and gentamicin (RPMI-FBS). Monocytes were purified from cytophoresis packs obtained from normal volunteers, as described (Shen, L. et al. (1986) Clin. Exp. Immunol. 65:387). Briefly, cells from cytophoresis packs were spun on Ficoll-Hypaque and the interface layer was collected. After three washes in RPMI, the cells were resuspended in RPMI-FBS at 5 X 10⁷/ml in 15 ml polypropylene tubes and were rotated at 8 rpm for 1 hr at 4°C to induce monocyte clumping. The clumped cells were sedimented on ice at 1 X G for 15 to 30 min, the supernatant was removed, and the cells (in 2 ml of medium) were then carefully layered onto an equal volume of ice-cold FBS. After sedimentation through the FBS for 20 min at 4°C, the lower phase contained 60 to 95% monocytes, the remainder being lymphocytes. Monocytes were washed twice in RPMI-FBS, were brought to 2 X 10⁶/ml in RPMI-FBS, and then were assayed. In some experiments, U937 cells (5 X 10⁵/ml or monocytes (2 X 10⁶/ml) were cultured for 18 to 24 hr in RPMI-FBS supplemented with 300 international reference units (IRU)/ml) of recombinant human interferon-γ (Genetech, San Francisco, CA).

### Target Cells.

HL-60 leukemia cells (ATCC CCL 240) were labeled for 1 hr at 37°C with 200 µCi of ⁵¹Cr sodium chromate in normal saline (New England Nuclear, Boston, MA). Cells were washed three times in medium 199-10% FBS before use.

### Antibody-Dependent Cellular Cytotoxicity (ADCC)

Equal volumes (50 µl) of ⁵¹Cr-labeled target cells at 5 x 10⁵/ml, effector cells at various effector to target ratios, and heteroantibodies at the concentrations indicated were mixed in roundbottomed microtiter wells. All tests were conducted in triplicate. Controls for the effects of heteroantibodies alone, and effector cells alone, were included in all experiments. Maximal lysis was obtained by the addition of 100 µl of 2% sodium dodecyl sulfate in water to 50 µl of CE. Plates were incubated for 18 hr at 37°C, after which 50% of the supernatant was removed and then counted for release of ⁵¹Cr. Percent cytotoxicity was calculated at 100 x (counts released with effectors + antibody) - (counts released with effectors alone) + (maximum lysis - spontaneous release). The results were expressed as mean ± standard deviation of triplicates.

### Cellular Heteroconjugates.

Target cells were coated for 2 hr at 4°C with heteroantibodies at the concentrations indicated, were washed three times, and were adjusted to 2 x 10⁷ cells/ml. Equal volumes (50 µl) of targets and effectors (2 x 10⁶/ml) were mixed by gentle rotation. for 1 hr at 4°C, and then allowed to settle for 1 hr on ice. The supernatant was removed and the cells were gently resuspended in 100 µl of acridine orange and examined in a hemocytometer by using incident light and UV. Effector cells (200) in duplicate samples were scored for attachment to one or more CE target cells.

### Microtiter Binding Assay

A monolayer of target cells was incubated in a microtitre plate well at 4°C with the heteroantibody construct. Unbound heteroantibodies were removed in a wash step. MTT labelled effector cells were added. MTT was then dissolved in isopropanol and a reading was taken using an ELISA reader at A 570.

### Results

The ability of the bispecific heteroantibody to mediate attachment of human monocytes to tumor target cells was confirmed in a microtiter well assay using MTT labelled monocytes and THP-1 human monocytic leukemia (ATCC TIB 202) or SKBR-3 breast carcinoma (ATCC HTB 30) target cells.

The ability of the heteroantibody to mediate killing of HL-60 promyelocytic leukemia cells was studied in the ADCC assay. Monocytes alone caused minimal killing (5-20%), monocytes plus bispecific heteroantibody caused moderate killing (20-50%), and monocytes plus bispecific heteroantibody plus human serum resulted in maximal killing (50-80%).

## Claims

1. A composition comprising a heteroantibody comprising an antibody (or fragment thereof) which binds a cell surface antigen, and an antibody (or fragment thereof) which binds the high affinity Fc-gamma receptor of an effector cell, the binding of which to an effector cell is not blocked by human immunoglobulin G, wherein the heteroantibody is capable of inducing complement mediated and effector-cell-mediated cell lysis and is in conjunction with:
(a) human complement; or
(b) human serum.

2. A composition comprising a target-specific effector cell comprising:
(i) an effector cell expressing high affinity receptor for the Fc portion of IgG; and
(ii) a heteroantibody comprising an antibody (or fragment thereof) which binds a cell surface antigen, and an antibody (or fragment thereof) bound to an epitope of said Fc receptor that is outside of the ligand binding domain of the receptor such that the binding is not blocked by human immunoglobulin G, wherein the target-specific cell is capable of inducing complement mediated and effector-cell-mediated cell lysis and is in conjunction with: (a) human complement; or (b) human serum.

3. The composition of claim 1 or claim 2 for use in therapy, for example in the complement-mediated lysis of cells in vivo or the treatment of conditions requiring complement-mediated cell lysis.

4. The composition of any one of claims 1-3 wherein the antibody which binds the cell surface antigen is an IgM antibody.

5. The composition of any one of the preceding claims wherein the cell surface antigen is the CD15 cell surface antigen.

6. The composition of any one of the preceding claims wherein the antibody (or fragment thereof) which binds the cell surface antigen and the antibody (or fragment thereof) which binds the receptor are linked by a disulphide bridge.

7. The composition of any one of the preceding claims wherein:
(a) the antibody (or fragment thereof) which binds the Fc-γ receptor is a monoclonal antibody that is produced by the hybridoma having ATCC accession number HB 9469; or
(b) the antibody fragment which binds the high affinity Fc-γ receptor is an Fab fragment of the monoclonal antibody produced by the hybridoma having ATCC accession number HB 9469; or
(c) the antibody which binds the high affinity Fc-γ receptor on an effector cell binds the Fc-γ receptor expressed on a human cell selected from the group consisting of monocytes, macrophages, neutrophils, and eosinophils; or
(d) the heteroantibody comprising an antibody (or fragment thereof) which binds the cell surface antigen binds the CD15 antigen on a CD-15-expressing cell selected from myeloid leukaemia, lung small cell carcinoma, colon carcinoma, and breast carcinoma.

8. The composition of any one of claims 2 or 3-7 (when dependent on claim 2) wherein the effector cell is a human cell selected from monocytes, macrophages, neutrophils or eosinophils.

9. Use of a composition as defined in any one of the preceding claims for the manufacture of a medicament for use in therapy, wherein the therapy is characterised by:
(a) administration of the heteroantibody or effector cell together with human complement or human serum; and
(b) complement-mediated lysis of cells or the treatment of conditions requiring complement-mediated cell lysis.

## Patentansprüche

1. Zusammensetzung, die einen Heteroantikörper mit einem Antikörper (oder dessen Bruchstück), der ein Zelloberflächen-Antigen bindet, und einen Antikörper (oder dessen Bruchstück) umfaßt, der den Hochaffinitäts-Fc-gamma-Rezeptor einer Effektorzelle bindet, dessen Bindung an eine Effektorzelle nicht durch menschliches Immunglobulin G blockiert wird, wobei der Heteroantikörper zur Induktion einer Komplement-vermittelten und Effektorzell-vermittelten Zell-Lyse in der Lage ist und mit:
(a) menschlichem Komplement, oder
(b) menschlichem Serum
in Verbindung ist.

2. Zusammensetzung mit einer zielspezifischen Effektorzelle, die folgendes umfaßt:
(i) eine Effektorzelle, die einen Hochaffinitäts-Rezeptor für den Fc-Anteil von IgG exprimiert, und
(ii) einen Heteroantikörper, der einen Antikörper (oder dessen Bruchstück), der ein Zelloberflächen-Antigen bindet, und einen Antikörper (oder dessen Bruchstück) umfaßt, der an ein Epitop des Fc-Rezeptors gebunden ist, das außerhalb der Liganden-Bindungs-Domäne des Rezeptores ist, derartig, daß die Bindung durch menschliches Immunglobulin G nicht blockiert wird, wobei die zielspezifische Zelle zur Induktion einer Komplement-vermittelten und Effektorzell-vermittelten Zell-Lyse in der Lage ist und mit: (a) menschlichem Komplement, oder (b) menschlichem Serum in Verbindung ist.

3. Zusammensetzung nach Anspruch 1 oder 2 zur Verwendung in der Therapie, beispielsweise bei der Komplement-vermittelten Lyse von Zellen in vivo oder bei der Behandlung von Beschwerden, die eine Komplement-vermittelte Zell-Lyse erfordern.

4. Zusammensetzung nach einem der Ansprüche 1 - 3, bei der der Antikörper, der das Zelloberflächen-Antigen bindet, ein IgM-Antikörper ist.

5. Zusammensetzung nach einem der vorhergehenden Ansprüche, bei der das Zelloberflächen-Antigen das CD 15- Zelloberflächen-Antigen ist.

6. Zusammensetzung nach einem der vorhergehenden Ansprüche, bei der der Antikörper (oder dessen Bruchstück), der das Zelloberflächen-Antigen bindet und der Antikörper (oder dessen Bruchstück), der den Rezeptor bindet, durch eine Disulfid-Brücke verbunden sind.

7. Zusammensetzung nach einem der vorhergehenden Ansprüche, bei der:
(a) der Antikörper (oder dessen Bruchstück), der den Fc-γ-Rezeptor bindet, ein monoklonaler Antikörper ist, der von der Hybridzelle erzeugt ist, die die ATCC-Zugangsnummer HB 9469 hat; oder
(b) das Antikörper-Bruchstück, das den Hochaffinitäts-Fc-γ-Rezeptor bindet, ein Fab-Bruchstück des von der Hybridzelle erzeugten monoklonalen Antikörpers ist, die die ATCC-Zugangsnummer HB 9469 hat; oder
(c) der Antikörper, der den Hochaffinitäts-Fc-γ-Rezeptor auf einer Effektorzelle bindet, den auf einer menschlichen Zelle exprimierten Fc-γ-Rezeptor bindet, wobei die Zelle aus der Gruppe ausgewählt ist, die aus Monozyten, Makrophagen, Neutrophilen und Eosinophilen besteht;
oder
(d) der Heteroantikörper, der einen Antikörper (oder dessen Bruchstück) umfaßt, der das Zelloberflächen-Antigen bindet, das CD 15-Antigen auf einer CD 15-exprimierenden Zelle bindet, die aus myeloischer Leukämie, kleinzelligem Lungenkarzinom, Kolonkarzinom und Mammakarzinom ausgewählt ist.

8. Zusammensetzung nach einem der Ansprüche 2 oder 3 - 7 (wenn sie von Anspruch 2 abhängig sind),
bei der die Effektorzelle eine menschliche Zelle ist, die aus Monozyten, Makrophagen, Neutrophilen oder Eosinophilen ausgewählt ist.

9. Verwendung einer wie in einem der vorhergehenden Ansprüche definierten Zusammensetzung zur Herstellung eines Medikaments zur Verwendung in der Therapie, wobei die Therapie durch folgendes gekennzeichnet ist:
(a) Verabreichung des Heteroantikörpers oder der Effektorzelle zusammen mit menschlichem Komplement oder mit menschlichem Serum, und
(b) Komplement-vermittelte Lyse von Zellen oder die Behandlung von Beschwerden, die eine Komplement-vermittelte Zell-Lyse erfordern.

## Revendications

1. Composition comprenant un hétéro-anticorps comprenant un anticorps (ou un fragment de ce dernier) qui se lie à un antigène de surface d'une cellule et un anticorps (ou un fragment de ce dernier) qui se lie au récepteur Fc-gamma de forte affinité d'une cellule effectrice, la liaison de l'anticorps à la cellule effectrice n'étant pas bloquée par l'immunoglobuline G humaine, dans laquelle l'hétéro-anticorps est capable d'induire une lyse cellulaire dont la médiation est assurée par le complément et dont la médiation est assurée par la cellule effectrice et est associé:
(a) au complément humain; ou
(b) à du sérum humain.

2. Composition comprenant une cellule effectrice apte à être ciblée de manière spécifique, comprenant:
(i) une cellule effectrice exprimant un récepteur de forte affinité pour la portion Fc de la IgG; et
(ii) un hétéro-anticorps comprenant un anticorps (ou un fragment de ce dernier) qui se lie à un antigène de surface d'une cellule et un anticorps (ou un fragment de ce dernier) lié a un épitope dudit récepteur Fc qui est situé à l'extérieur du domaine de liaison du récepteur à un ligand de telle sorte que la liaison n'est pas bloquée par l'immunoglobuline G humaine, dans laquelle la cellule apte à être ciblée de manière spécifique est capable d'induire une lyse cellulaire dont la médiation est assurée par le complément et dont la médiation est assurée par la cellule effectrice et est associée: (a) au complément humain; ou (b) à du sérum humain.

3. composition selon la revendication 1 ou 2 à utiliser en thérapie, par exemple dans la lyse de cellules in vivo dont la médiation est assurée par le complément ou dans le traitement d'affections nécessitant une lyse cellulaire dont la médiation est assurée par le complément.

4. Composition selon l'une quelconque des revendications 1 à 3, dans laquelle l'anticorps qui se lie à l'antigène de surface d'une cellule est un anticorps IgM.

5. Composition selon l'une quelconque des revendications précédentes, dans laquelle l'antigène de surface d'une cellule est l'antigène de surface CD15.

6. Composition selon l'une quelconque des revendications précédentes, dans laquelle l'anticorps (ou un fragment de ce dernier) qui se lie à l'antigène de surface d'une cellule et l'anticorps (ou un fragment de ce dernier) qui se lie au récepteur sont reliés par un pont disulfure.

7. composition selon l'une quelconque des revendications précédentes, dans laquelle:
(a) l'anticorps (ou un fragment de ce dernier) qui se lie au récepteur Fc-γ est un anticorps monoclonal qui est produit par l'hybridome possédant le numéro matricule HB 9469 de ATCC; ou
(b) le fragment d'anticorps qui se lie au récepteur Fc-γ de forte affinité est un fragment Fab de l'anticorps monoclonal produit par l'hybridome possédant le numéro matricule HB 9469 de ATCC; ou
(c) l'anticorps qui se lie au récepteur Fc-γ de forte affinité sur une cellule effectrice se lie au récepteur Fc-γ exprimé sur une cellule humaine choisie parmi le groupe constitué par des monocytes, par des macrophages, par des polynucléaires neutrophiles et par des poiynucléaires éosinophiles; ou
(d) l'hétéro-anticorps comprenant un anticorps (ou un fragment de ce dernier) qui se lie à l'antigène de surface d'une cellule se lie à l'antigène CD15 sur une cellule exprimant CD15 que l'on retrouve dans des affections choisies parmi la leucémie myélogène, le cancer pulmonaire à petites cellules, le cancer du côlon et le cancer du sein.

8. Composition selon l'une quelconque des revendications 2 ou 3-7 (lorsqu'elles dépendent de la revendication 2), dans laquelle la cellule effectrice est une cellule humaine choisie parmi des monocytes, des macrophages, des polynucléaires neutrophiles ou des polynucléaires éosinophiles.

9. Utilisation d'une composition telle que définie dans l'une quelconque des revendications précédentes pour la fabrication d'un médicament à utiliser en thérapie, dans laquelle la thérapie se caractérise par:
(a) l'administration de l'hétéro-anticorps ou de la cellule effectrice conjointement avec le complément humain ou avec du sérum humain; et
(b) la lyse cellulaire dont la médiation est assurée par le complément ou le traitement d'affections nécessitant une lyse cellulaire dont la médiation est assurée par le complément.
